# EUROPEAN PATENT SPECIFICATION

(11) **EP 1 996 157 B1**
(45) Date of publication and mention of the grant of the patent: **23.09.2015**
(21) Application number: 07736693.8
(22) Date of filing: 15.03.2007
(51) Int. Cl.: A61K 9/00, A61K 9/107

(54) **AN OPHTHALMIC PHARMACEUTICAL COMPOSITION CONTAINING AMPHIPHILIC POLYASPARTAMIDE COPOLYMERS**
OPHTALMISCHE PHARMAZEUTISCHE ZUSAMMENSETZUNG ENTHALTEND AMPHIPHILEN POLYASPARTAMID COPOLYMERE
COMPOSITION PHARMACEUTIQUE OPHTALMOLOGIQUE CONTENANT DES COPOLYMÈRES POLYASPARTAMIDE AMPHIPHILES

(30) Priority: 17.03.2006 IT MI20060494
(43) Date of publication of application: 03.12.2008
(73) Proprietor: Sifi S.p.A., 95020 Lavinaio-Aci Sant'Antonio (CT) (IT)
(72) Inventor: GIAMMONA, Gaetano c/o Universita degli Studi di Palermo, I-90123 Palermo (IT); CAVALLARO, Gennara c/o Universita degli Studi di Palermo, I-90123 Palermo (IT); LICCIARDI, Mariano c/o Universita degli Studi di Palermo, I-90123 Palermo (IT); CIVIALE, Claudine, I-95020 Lavinaio Aci Sant'Antonio, CATANIA (IT); PALADINO, Grazia Maria, I-95020 Lavinaio Aci Sant'Antonio, CATANIA (IT); MAZZONE, Maria Grazia, I-95020 Lavinaio Aci Sant'Antonio, CATANIA (IT)
(74) Representative: Long, Giorgio
(86) International application number: PCT/IT2007/000188
(87) International publication number: WO 2007/108031

(56) References cited:
- WO-A-03/002096
- WO-A-03/082303
- CALICETI P. ET AL.: "SYSNTHESIS AND BIOPHARMACEUTICAL CHARACTERISATION OF NEW POLY(HYDROXYETHYLASPARTAMIDE) COPOLYMERS AS DRUG CARRIER" BIOCHEMICA ET BIOPHYSICA ACTA, vol. 1528, no. 2-3, 3 October 2001 (2001-10-03), pages 177-186, XP002455058
- CAVALLARO, GENNARA ET AL: "Tamoxifen-loaded polymeric micelles: Preparation, physico-chemical characterization and in vitro evaluation studies" 2004, MACROMOLECULAR BIOSCIENCE , 4(11), 1028-1038 CODEN: MBAIBU; ISSN: 1616-5187 , XP002455144 abstract
- PITARRESI G.,CAVALLARO G. ET AL.: "Soluble and water-swellable polyaminoacidic derivatives as drug carriers" RECENT RES. DEVEL. MACROMOL., vol. 6, 2002, pages 1-35, XP008084853 kerala, India

## Description

### FIELD OF THE INVENTION

The present invention relates to the use of amphiphilic graft-type copolymers of polyaspartamide for the ophthalmic administration of drugs, such as for example steroid and non-steroid anti-inflammatory agents, antimicrobial agents such as aminoglycosides, macrolides, cephalosporin, tetracycline, quinolones, penicillin, beta-lactams, anti-glaucoma agents such as prostaglandins, prostamides, alpha- and beta-blockers, inhibitors of carbonic anhydrase, cannabinbids, antiviral agents, diagnostic agents, anti-angiogenic agents, antioxidants. At appropriate concentrations, in aqueous environments, such copolymers can form polymeric micelles capable of incorporating drugs.

### STATE OF THE ART

Micelles are association colloids obtained by the auto-aggregation of amphiphilic molecules (surfactants) above a certain concentration, (critical micelle concentration, CMC). Polymeric micelles represent a separate class of micelles, constituted by amphiphilic copolymers, i.e. containing hydrophilic and hydrophobic monomer units. These two types of monomer units can be organised in various ways inside the polymer chain, giving rise to random copolymers (if the sequence is entirely random), block copolymers (if all the units of a certain type are linked together and distinguished from those with different polarity) and graft copolymers (if opposite polarity chains are bound in random order to the main polymer backbone). The amphiphilic nature of these copolymers allows the individual macromolecules to behave as surface acting agents (surfactants) in solution, or rather to organise themselves into micellar-type aggregates above a certain concentration (CMC, or critical aggregation concentration, CAC). In water, the micellar structure is core-shell type, wherein the hydrophobic domains associate internally (core), while the soluble domains form an outer layer (shell). Just like the situation that occurs for low molecular weight surfactants, the driving force for spontaneous association is the reduction of free energy of the system resulting from the removal of apolar residues from the aqueous environment, with the formation of a hydrophobic "core", stabilised by the hydrophilic residues exposed to the aqueous environment.

Polymeric micelles have very variable morphology, and the factors influencing such aspects include the composition of the copolymer, its concentration, and the solvent medium used for preparation; they are normally considered to be spherical or ellipsoidal aggregates with dimensions comprised of between 5 and 100 nm. In any case, even their dimensions depend on several parameters including among others, the type and molecular weight of copolymer, the hydrophobic/hydrophilic ratio and the number of macromolecules (unimers) per micellar aggregate. Generally, the micelles formed by graft-type copolymers are smaller than those formed by block-type copolymers, since they can even be constituted by a single polymer chain and thus have a lower aggregation number. Furthermore, polymeric micelles are characterised by significant thermodynamic stability, expressed by the standard free energy value associated with the micellisation (micelle-forming) process, which is dependent on the CAC value, and high kinetic stability, which is dependent on the rate of dissociation (Kd) of the unimers following dilution; indeed, even following dilution to concentrations less than the CAC value, preformed micelles can exist for a sufficiently long period of time, in the order of hours or days, allowing the aggregate to perform its function.

Polymeric micelles have been suggested for making colloidal systems as carriers for drugs and parenterally-administered diagnostic contrast agents.

The patent WO 03082303 (ABBOTT LAB) 9 October 2003 discloses (see page 26, Paragr. 2-page 27 line 2 and claim 23) a micelle composition with poly(ethyleneglycol)-block-poly(N-hexyl-L-aspartamide) -stearate ester, which is useful for reducing the toxicity of polyene antibiotic, particularly amphotericin B. Also for inhibiting pathogenic fungi. This composition is administered parenterally (e.g. intravenous injection or intravenous perfusion), intradermally, intratumorally, intramuscularly, intraperitoneally, mucosally, orally or topically. The dry micelles are reconstituted in a pharmaceutically acceptable carrier such as sterile physiological saline or a sterile dextrose solution.

The article, Biochimica et Biophysica Acta, vol.1528, Issues 2-3, 3 October 2001, pages 177-186, 'Synthesis and biopharmaceutical characterisation of new polyhydroxyethylaspartamide copolymers as drug carriers', by Caliceti P. et al., discloses (see page 4 paragr. 2-5 und page 6 paragr.4-page 8 paragr. 6) PHEA-PEG(2000-5000) - C16 and PHEA-PEG(2000-5000) were obtained by partial aminolysis of PSI and used as drug carrier.

The drug or diagnostic agent may be incorporated in the micelles by means of simple physical interaction, principally with the hydrophobic portions of the micellar "core" or by means of covalent bonding with functional groups of the constituent copolymer. The release mechanism of the drug from polymeric micelles is strictly dependent on the type of type of bond existing between the drug and the micellar "core". In other words, for drugs incorporated by means of physical bonds, release will be controlled by the rate of diffusion of such molecules in the micellar "core" and the micelle-unimer equilibrium; in the case of drugs bound to the micellar core by means of covalent bonds, release of the drug will be dependent not only on the dissociation equilibrium of the micelles, but also on the hydrolysis of the drug-polymer bonds, together with the penetration process of the water into the micellar core.

Due to their amphiphilic nature, unimers are also capable of interacting with amphiphilic biological molecules, such as the phospholipids of cell membranes, and consequently modifying the permeability of the same.

To date, the ophthalmic administration of drugs is exclusively limited to molecules administered for locally acting diagnostic or therapeutic purposes, which are administered by dropping them or in any case applying them as they are, or by means of aqueous or gel-based systems or lipid-based systems, to the precorneal area. The drug can be eliminated from this ocular region by means of various mechanisms, including lachrymal turnover, drainage of the instilled liquid, binding to lachrimal proteins, enzymatic degradation of the drug, non-productive absorption (i.e. loss predominantly due to absorption by non-corneal tissues) or be absorbed transcorneally, conjunctively or sclerally, and reach the inner eye structures where it is called on to play its therapeutic or diagnostic action.

However, in general, the ocular bioavailability of topically applied drugs is rather low; indeed, ocular absorption is severely limited by, in addition to the previously mentioned factors (which cause the elimination of the drug), the limited area available for absorption and by the protective mechanisms which ensure correct eye function (including the blink reflex).

Such factors, taken together, generally mean that the quantity of drug reaching the aqueous humor and the inner eye structures is very low, generally no more than 1-10% of the instilled dose. Furthermore, the fraction of drug made available by the various routes (naso-lachrymal canal, conjunctive, gastrointestinal tract etc.) at the systemic level, can give rise to undesired side effects, the extent of which is strictly correlated with the percentage of drug undergoing systemic absorption which, in turn, depending on the drug, can vary from 3 to 80% of the dose instilled.

Hence, the idea underlying the present invention arises from the need to develop ophthalmic formulations capable of increasing the transcorneal and/or transconjunctival absorption of drugs, and hence increase the fraction of drug capable of penetrating the cornea e/o the conjunctive to reach the inner eye structures; this also occurs while maintaining the structural and functional characteristics of the same unaltered.

In particular, the approach used in the present invention has been that of using polyaspartamide-based polymer micelles to obtain formulations capable of increasing the ocular bioavailability of drugs for ocular use (such as for example steroidal anti-inflammatory agents, antimicrobial drugs, anti-glaucoma agents, antihypertensives, diagnostic agents, antiviral agents, anti-angiogenic agents, antioxidants). Such micelles have been shown to be capable of increasing the ocular bioavailability of drugs by 60% with respect to the drug being administered as it is, thanks to increased transcorneal permeability; furthermore, such carriers have shown optimal ocular tolerability *in vivo* and low cellular toxicity in the *in vitro* and *in vivo* model systems used.

Hence, the use of such carriers allows increased ocular bioavailability of drugs, while consequently reducing side effects due to the systemic absorption of the same.

### BRIEF DESCRIPTION OF THE FIGURES

The invention will now be clearly exemplified with reference to the appended figures, wherein:
- Figure 1 represents photomicrographs of SIRC cells (cells derived from rabbit cornea), comparing the morphology of untreated cells with those treated in accordance with the invention;
- Figure 2 represents a graph of cellular recovery (and hence activity) following inclusion of the pharmacological carrier of the invention;
- Figure 3 represents a graph of dexamethasone concentration in aqueous humor following administration in the eyes of rabbits.

### SUMMARY OF THE INVENTION

The object of the present invention is that of creating novel ophthalmic formulations of steroidal and non-steroidal anti-inflammatory drugs, antimicrobial agents, anti-glaucoma agents, antiviral agents, anti-angiogenic agents, antioxidants, and diagnostic agents physically incorporated inside micellar systems constituted by amphiphilic polyaspartamide copolymers with the scope of increasing the quantity of bioavailable drug at the ocular level with respect to that which can be obtained by administering the free drug; this is with the aim of reducing the dose to be administered and the number of administrations, and thus obtain a therapeutic advantage. A further object of the present invention is that of providing systems which will allow less non-productive absorption of the drugs, with consequent reduction of the undesired systemic side effects which are correlated with the use of the aforementioned drugs when administered in their native states.

Another object of the present invention is that of using a family of polyaspartamide-based polymer surfactants, endowed with high compatibility, ease of reproducible manufacture at high yields and low cost, and modulability in terms of hydrophobic and hydrophilic chain content, in the production of ophthalmic formulations.

### DESCRIPTION OF THE INVENTION

The main object of the present invention is to provide an ophthalmic pharmaceutical composition according to claim 1, and this ophthalmic formulation is capable of increasing the ocular bioavailability of drugs (steroidal and non-steroidal anti-inflammatory agents, antimicrobial agents, anti-glaucoma agents, antiviral agents, anti-angiogenic agents, antioxidants, diagnostic agents) using a family of polymeric surfactants constituted by graft-type amphiphilic polyaspartamide copolymers of (α,β-poly(N-2-hydroxyethyl-DL-aspartamide)) (PHEA).

Graft copolymers of polyaspartamide (PHEA) containing different percentages of the hydrophilic chain (polyethyleneglycol, PEG, with mean molecular weight comprised of between 750 and 20000 Da) and/or hydrophobic chain (Cₙ, with 4 ≤ n ≤ 20), respectively (PHEA-PEGx-Cₙ and PHEA-Cₙ wherein x = the mean molecular weight of the PEG and n = the number of carbon atoms in the introduced alkylamine chain) have been prepared, characterised and evaluated from the viewpoint of ocular tolerability *in vivo,* cytotoxicity and cell permeability in *vitro* and in *vivo* following ocular administration. Preferably, the mean molecular weight of the PEG chain is comprised of between 2000 and 10000 Da, more preferably, it is 5000 Da, while the number of carbon atoms in the alkylamine chains is 12 ≤ n ≤ 18, more preferably n = 16. Preparation of the aforementioned copolymers has been carried out by means of sequential reactions envisaging the insertion of a PEG chain (mean molecular weight comprised of between 750 and 20000 Da and/or Cₙ alkyl chain with 4 ≤ n ≤20) into the polyaspartamide structure through the use of partial aminolysis reactions with organic amines.

For the α,β-poly(N-2-hydroxyethyl-DL-aspartamide-alkylaminamine (PHEA-Cₙ) copolymers, the following reactions have been used in sequence:
- partial aminolysis of polysuccinimide (PSI) (obtained by the thermal condensation of D,L-aspartic acid, at a temperature of between 100-320°C, preferably between 150 and 250°C, more preferably between 180-210°C, at a pressure of between 1-10⁻² mmHg and in the presence of phosphoric acid as catalyst) with alkylamine (C*ₙ*, with 4 ≤ n ≤ 20, preferably with 12 ≤ n ≤ 18, more preferably n = 16) in dimethylformamide (DMF) solution, at a constant temperature of between 20 and 100°C, preferably between 40 and 80°C, more preferably 60°C, using a molar ratio between the moles of Cₙ and repetitive PSI units of between 0.01-0.6 for an appropriate length of time (between 1 and 15 hours) so as to obtain PSI-Cₙ copolymers;
- subsequent complete aminolysis of the PSI-Cₙ copolymers obtained with an excess of ethanolamine in DMF solution for a suitable period of time (between 0.5 and 10 hours). Upon completion of the reaction time, the products have been recovered by precipitation in ethyl ether, centrifuged, washed several times (for example between 3 and 5 times) with acetone, dried under vacuum and purified by exhaustive dialysis against double-distilled water.

The yields have been in the 94-100 % w/w range with respect to the starting quantity of PSI. Each product obtained has been characterised spectrophotometrically. The molar percentage of alkylamine chains present in the PHEA-Cn copolymers, as determined by NMR, is comprised of between 0.5 and 20%. The copolymers obtained are freely soluble in water.

For the α,β-poly(N-2-hydroxyethyl-DL-aspartamide-poly(ethyleneglycol)-alkylamine (PHEA-PEGx-Cₙ) copolymers, 3 reactions have been carried out in sequence:
- partial aminolysis of polysuccinimide (PSI) (obtained as specified above) with O-(2-aminoethyl)-O'-methylpolyethylene (NH₂-PEGₓ-OCH₃) (with x indicating the mean molecular weight, comprised of between 750 and 20000 Da, with preferred molecular weight comprised of between 2000 and 10000 Da, particularly preferred mean molecular weight of 5000 Da) in dimethylformamide (DMF) solution, at constant temperature between 20 and 100°C, preferably between 40 and 80°C, more preferably 60°C, using a molar ratio between the moles of NH₂-PEGₓ-OCH₃ and repetitive PSI units of between 0.01 and 1, for an appropriate period of time between 1 and 40 hours, in order to give PSI-PEGₓ copolymers;
- subsequent partial aminolysis of the polysuccinimide-poly(ethylglycol) (PSI-PEGₓ) copolymers (obtained as specified above) with alkylamine (Cₙ, with 4 ≤ n ≤ 20, preferably with 12 ≤ n ≤ 18, particularly preferred n = 16) in dimethylformamide (DMF) solution, at constant temperature between 20 and 100°C, preferably between 40 and 80°C, more preferably 60°C, using a molar ratio between the moles of Cₙ and repetitive PSI units of between 0.01-0.80 for an appropriate period of time between 1 and 15 hours, in order to give PSI-PEGₓ-Cₙ copolymers;
- subsequent complete aminolysis of the PSI-PEGₓ-Cₙ copolymers obtained with an excess of ethanolamine in DMF solution for a suitable period of time comprised of between 1 and 5 hours.

Upon completion of the reaction time, the products have been recovered by precipitation in ethyl ether, centrifuged, treated several times with acetone, dried under vacuum and purified by exhaustive dialysis against double-distilled water.

The yields have been in the 94-100% w/w range with respect to the starting quantity of PSI. Each product obtained has been characterised spectrophotometrically. The molar percentage of poly(ethyleneglycol) (PEG) chain and alkylamine chain present in the PHEA-PEGₓ-Cₙ copolymers, as determined by NMR, is comprised of between 0.5 and 30% and 0.5 and 20% respectively. The copolymers obtained are freely soluble in water.

The copolymers obtained by means of the present invention have been characterised in terms of molecular weight and polydispersity index by means of Size Exclusion Chromatography (SEC). The mean measured molecular weights determined in water are comprised of between: 10 KDa and 150 KDa; the polydispersity indices have been determined to be between 1.1 and 2.

Purely by way of example, the present description reports the results relating to copolymers containing PEG with mean molecular weight, of 5000 Da and C₁₆ (hexadecylamine) hydrophobic Cₙ chains, with lipophilic molecules such as steroids and hydrophilic molecules such as aminoglycosides. The studies carried out support and suggest the use of all the copolymers tested to vehicularise drugs (such as for example, steroidal and non-steroidal anti-inflammatory agents, antimicrobial agents, anti-glaucoma agents, antiviral agents, anti-angiogenic agents, antioxidants, diagnostic agents) for ophthalmic administration.

The copolymers described in the present invention have been subjected to Langmuir Trough (LT) studies, so as to obtain information on the complexing capacity for drugs (for example dexamethasone alcohol) of the polymeric micelles formed by them, and Micellar Affinity Capillary Electrophoresis (MACE) with the aim of obtaining information on the interaction of an aqueous phase containing the copolymers in question and a lipid monolayer as a membrane model.

The LT studies have highlighted that, unlike the parent polymer (PHEA), there is interaction between such copolymers and Dipalmitoylphosphatidylcholine lipid monolayers, and that this predominantly occurs between the hexadecylamine chains of the copolymers and the nonpolar portion of the lipid monolayer.

The MACE studies have shown for example that, unlike PHEA, copolymers containing hexadecylamine chains (PHEA-C₁₆ and PHEA-PEG5000-C₁₆) have the capacity to complex drugs, such as for example dexamethasone, and that said capacity also depends on the temperature.

The micellar systems described in the present invention have been subjected to permeability studies on layers of bovine conjunctival epithelial cells (BCEC layers). The effect of the aforementioned systems on paracellular transport has been evaluated using carboxyfluorescein, and the conjunctival permeability of steroidal anti-inflammatory drugs and aminoglycoside antimicrobial agents has been evaluated using the same model, purely by way of example in order to compare a class of lipophilic molecules with respect to a class of hydrophilic molecules.

By way of example, we report that the PHEA-PEG5000-C₁₆ and PHEA-C₁₆ copolymers do not influence the paracellular transport of carboxyfluorescein, and in particular, the PHEA-PEG5000-C₁₆ derivative significantly increases the permeability of steroidal anti-inflammatory drugs, such as for example dexamethasone and aminoglycoside antimicrobial agents such as for example netilmycin, in the model used.

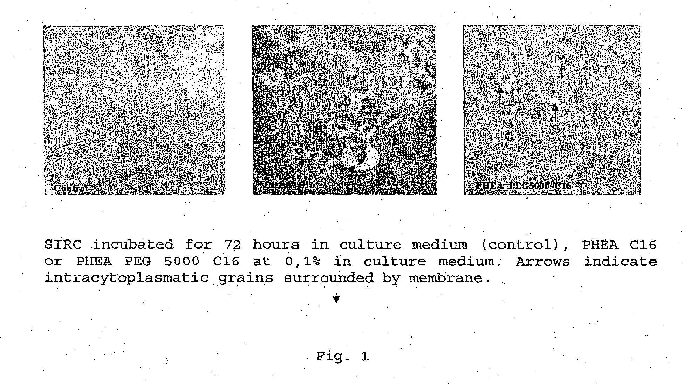

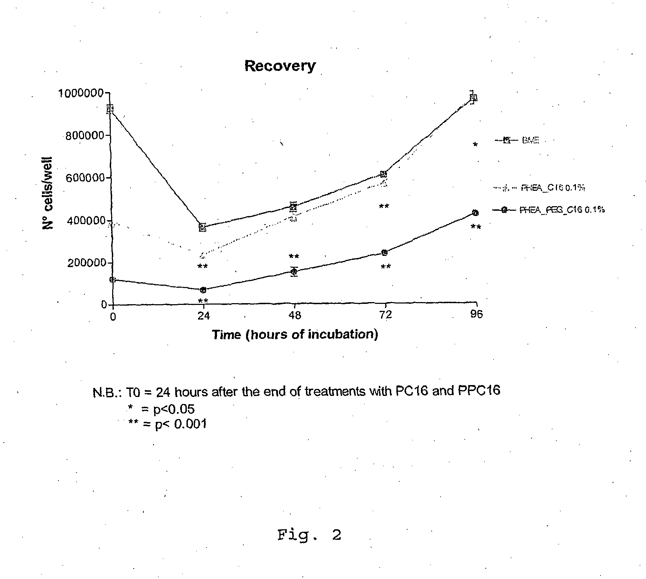

Studies of *in vitro* permeability across layers of bovine corneal epithelial cells have shown that all the copolymers tested increase the transcorneal permeability of steroidal anti-inflammatory drugs such as for example dexamethasone alcohol and dexamethasone phosphate ester. The copolymers forming the subject of the present invention have been subjected to *in vitro* biocompatibility studies with rabbit corneal cells (SIRC), in order to assess any potential cytotoxicity of such systems using light microscopy (morphological evaluation of the cell monolayer) or transmission electron microscopy (TEM). Cells incubated in growth medium containing PHEA-C₁₆ and PHEA-PEG5000-C₁₆ observed by TEM, show amorphic intracytoplasmic granules surrounded by plasma membrane, which can be attributed to invaginations containing the polymers (fig. 1). However, the cells show no signs of cellular or nuclear damage; the cellular cytoplasm shows ultrastructural characteristics similar to controls and well functioning mitochondria. The presence of the copolymers slows cellular metabolism, as demonstrated by metabolic viability assays (MTT tests), but the cells incorporating such substances into their cytoplasms are capable of metabolising them, and restoring normal metabolic activity following "recovery" with normal culture medium devoid of the polymeric systems (fig. 2).

The systems described in the present invention have been subjected to *in vivo* studies in rabbits with the aim of assessing the ocular tolerability of the polymer micelles with respect to control (BSS, Buffered Saline Solution) when administered into the ocular sac of the animal (Draize test); the studies have shown good ocular tolerability in all animals. Furthermore, such studies have not highlighted any clinical signs (normal weight increase curves and food and water consumption), indicating potential systemic absorption, for any of the systems tested with respect to the control.

Studies on the influence on the *in vivo* transcorneal transport of drugs, such as for example steroidal anti-inflammatory agents, have shown that PHEA-PEGx-Cn micelles result in a significant increase in the concentration of the drug in the aqueous humor with respect to the commercial formulation with an equal quantity of drug.

### EXAMPLE 1

The *in vivo* transcorneal transport experiments have been performed on male albino rabbits (Charles River) weighing 1.8-2.5 Kg. Prior to the experiment, the animals have been kept in standard cages with free access to food and water. The rabbits have been treated in accordance with the instructions published in "Guiding Principles in the Care and Use of Animals" (DHEW Publication, NIH 80-23) and the ARCO Resolution on the Use of Animals in Research.

Two dexamethasone alcohol formulations have been used for the study:
- Visumetazone^{®} (a commercial formulation of the drug, in a 0.1% w/v suspension);
- Micellar formulation (drug, at a final concentration of 0.1% w/v, incorporated inside PHEA-PEG5000-C16 micelles in isotonic phosphate buffer at pH 7.3).

The formulation of dexamethasone-containing PHEA-PEG₅₀₀₀-C₁₆ micelles has been prepared immediately prior to testing by mixing together the appropriate quantity of copolymer and drug into a homogeneous paste using modified Ringer's solution as a dispersant and subsequently adding a suitable volume of modified Ringer's solution in order to give a final dexamethasone concentration equal to 0.1% w/v, and a polymer concentration equal to 0.2% w/v.

The tests have been conducted by administering 50 µl of one of the previously described formulations into the conjunctival sac of rabbits.

At various times within the 30 to 180 minute range, samples of aqueous humor have been removed from the animals, following euthanasia. Said samples have been added to a suitable quantity of methanol in order to precipitate-out the protein components of the aqueous humor, then following centrifugation, the supernatant has been analysed by HPLC using a 4.6/125 mm Hypersil ODS 5 µm column as the stationary phase, with a H5ODS-120CS pre-column, and a 53%/47% gradient of 0.05 M (6.8 g/l) CH₃OH/KH₂PO₄, pH 6 as the mobile phase, at a flow rate of 1 ml/min, injection volume of 100 µl, and the eluate monitored at 242 nm.

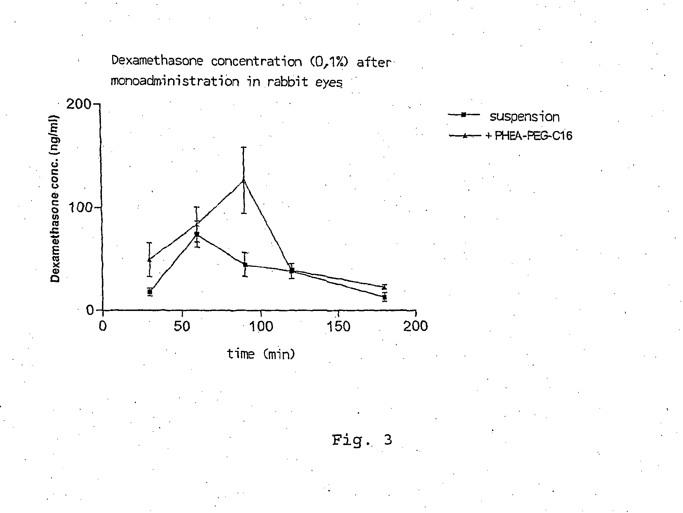

The results of said study, reported in Figure 3, show an increase in area under the curve (AUC) of approx. 60% using the formulation of PHEA-PEG₅₀₀₀-C₁₆ micelles (0.02%) containing dexamethasone alcohol (0.1%), with respect to the commercial formulation (0.1% Visumetazone suspension).

### EXAMPLE 2

The *in vitro* permeability studies have been conducted using a bovine conjunctival epithelial cell (BCEC) multilayer, which reproduces the organisation of the original tissue, as a model.

Two formulations have been used for the studies with netilmycin sulphate:
✔ Formulation of netilmycin sulphate in modified Ringer's solution, with a netilmycin base concentration of 0.3% w/v;
✔ Micellar formulation (netilmycin sulphate incorporated in PHEA-PEG₅₀₀₀-C₁₆ micelles in modified Ringer's solution, with a netilmycin base concentration of 0.3% w/v).

The netilmycin-containing formulation has been prepared immediately prior to testing by mixing together the appropriate quantity of copolymer and drug into a homogeneous paste using modified Ringer's solution as a dispersant and subsequently adding a suitable volume of modified Ringer's solution in order to give a final netilmycin base concentration equal to 0.3 % w/v.

The tests have been conducted by introducing 0.5 ml of one of the aforementioned formulations into the donor compartment and 1.5 ml of modified Ringers solution into the acceptor compartment; incubation lasted 120 minutes at 37 °C, with orbital shaking at 50 rpm. At subsequent times equal to 30, 60, 90 and 120 minutes, 500 µl samples have been removed from the acceptor compartment and subsequently replaced with fresh Ringer's solution. The removed samples have been analysed by HPLC, following derivatisation of the netilmycin with orthophthalaldehyde (OPA)to carry out the analytical determination. The derivatisation procedure has been conducted as reported hereinafter. To one part of the standard netilmycin solution (or solution removed from the acceptor compartment) are added three parts of OPA solution (prepared by dissolving 20 mg of OPA in 5 ml of methanol, diluting said solution in 90 ml of borate buffer pH 10.4 and adding 0.2 ml of thioglycolic acid) and one part of methanol. The solution thus obtained is kept at 60°C for 15 minutes, away from light. Each solution is then cooled rapidly by placing in an ice bath for 5 minutes and then placed in an autosampler set at 4°C prior to being analysed by HPLC. Under such conditions, the fluorescence of the netimycin-OPA derivative is stable for at least 3 hours, within which time, the sample has been analysed. HPLC analysis has been conducted using a 4.6/250 mm LUNA C₁₈ 5 µm column as stationary phase, with a H₅ODS-C₅ pre-column, and a 30%/70% gradient of 0.05 M (pH 7.3) CH₃CN/KH₂PO₄ as mobile phase, at a flow rate of 1 ml/min, an injection volume of 100 µl and monitoring the eluate at 330 nm.

Each formulation has been tested ten times on a similar number of BCEC multilayer samples.

Multilayer integrity has been tested by TEER measurements at the start of each experiment, after 30 and 60 minutes, and at the end of each experiment.

Two types of statistical test have been used to determine the level of significance of the data: analysis of variance and Student t-test.

It has been shown that the coefficient of permeability of the drug vehicularised in PHEA-PEG₅₀₀₀-C₁₆ micelles is equal to roughly twice that obtained with the free drug.

### Bibliography

1) Caliceti P., Quarta S. M., Veronese F. M., Cavallaro G., Pedone E. Giammona G. Biochim. Biophys. Acta, 1528 (2001) 177-186.
2) Cavallaro G., Licciardi M., Giammona G. , Caliceti P., Semenzato A., Salmaso S. J. Controlled Release, 89 (2003) 285-295.

## Claims

1. Pharmaceutical compositions for use in the treatment of ophthalmic diseases by local administration comprising one or more active ingredients selected from antimicrobial agents, anti-inflammatory agents, anti-glaucoma agents, antiviral agents, anti-angiogenic agents andantioxidants said active agent being included in a pharmaceutical vector constituted by polymer micelles comprising "graft" type copolymers of polyaspartamide (PHEA) containing hydrophilic polyethylene glycol (PEG) chains with a mean molecular weight comprised between 750 and 20000 Da and/or hydrophobic Cₙ alkyl chains, wherein 4 ≤ n ≤ 20..

2. Pharmaceutical compositions according to claim 1, further comprising one or more pharmaceutically acceptable excipients for ophthalmic administration.

3. Pharmaceutical compositions according to claim 2, wherein the excipients are selected from: bioadhesives, electrolytes (phosphates, chlorides), non-ionic isotonizing agents, surfactants, antioxidants, buffer systems.

4. The pharmaceutical compositions according to claim 1, wherein the mean molecular weight of the polyethylene glycol chains is equal or comprised between 2000 and 10000 Da, and the number of carbon atoms in the hydrophobic chains is 12 ≤ n ≤ 18.

5. Pharmaceutical compositions according to claim 1 or 4, wherein the hydrophilic chains are constituted by polyethylene glycol PEG with mean molecular weight comprised between 750 and 20000 Da and the lipophilic chains by aliphatic chains selected from the group consisting of: butylamine, pentylamine, hexylamine, heptylamine, octylamine decylamine, undecylamine, dodecylamine, tridecylamine, tetradecylamine, hexadecylamine, octadecylamine, didecylamine.

6. Pharmaceutical compositions according to claim 1 or 5, wherein the copolymers comprise PEG with mean molecular weight of 5000 Da and Cₙ hydrophobic chains wherein n is 16.

7. A pharmaceutical composition for local administration for use in the diagnosis of ophthalmic diseases comprising a diagnostic agent, said diagnostic agent being included in a pharmaceutical vector constituted by polymer micelles comprising "graft" type copolymers of polyaspartamide (PHEA) containing hydrophilic polyethylene glycol (PEG) chains with a mean molecular weight comprised between 750 and 20000 Da and/or hydrophobic Cₙ alkyl chains, wherein 4 ≤ n ≤ 20.

## Patentansprüche

1. Pharmazeutische Zusammensetzungen zur Verwendung bei der Behandlung von ophthalmischen Krankheiten durch lokale Verabreichung umfassend ein oder mehrere aktive Inhaltsstoffe ausgewählt aus antimikrobiellen Mitteln, antiinflammatorischen Mitteln, anti-Glaukom Mitteln, antiviralen Mitteln, antiangiogenen Mitteln, und Antioxidantien, wobei das aktive Mittel in einem pharmazeutischen Träger enthalten ist, der durch Polymer-Mizellen gebildet ist, die "Propf"-Typ-Copolymere von Polyaspartamid (PHEA) umfassen, die hydrophile Polyethylen-Glykol (PEG) Ketten mit einem mittleren Molekulargewicht umfassend zwischen 750 und 20000 Da und/oder hydrophobe Cₙ Alkyl-Ketten mit 4 ≤ n ≤ 20 beinhalten.

2. Pharmazeutische Zusammensetzungen nach Anspruch 1, die weiter ein oder mehrere pharmazeutisch annehmbaren Hilfsstoffe zur ophthalmischen Verabreichung umfassen.

3. Pharmazeutische Zusammensetzungen nach Anspruch 2, wobei die Hilfsstoffe ausgewählt sind aus: Bioadhäsiven, Elektrolyten (Phosphaten, Chloriden), nichtionischen isotonisierenden Mitteln, oberflächenaktiven Stoffen, Antioxidantien, Puffersystemen.

4. Die pharmazeutischen Zusammensetzungen nach Anspruch 1, wobei das mittlere Molekulargewicht der Polyethylen-Glykol-Ketten gleich oder umfassend zwischen 2000 und 10000 Da ist, und die Zahl der Kohlenstoffatome in den hydrophoben Ketten 12 ≤ n ≤ 18 ist.

5. Pharmazeutische Zusammensetzungen nach Anspruch 1 oder 4, wobei die hydrophilen Ketten durch Polyethylen-Glykol PEG mit einem mittleren Molekulargewicht umfassend zwischen 750 und 20000 Da und die lipophilen Ketten durch aliphatische Ketten ausgewählt aus der Gruppe bestehend aus: Butylamin, Pentylamin, Hexylamin, Heptylamin, Octylamin, Decylamin, Undecylamin, Dodecylamin, Tridecylamin, Tetradecylamin, Hexadecylamin, Octadecylamin, Didecylamin gebildet sind.

6. Pharmazeutische Zusammensetzungen nach Anspruch 1 oder 5, wobei die Copolymere PEG mit einem mittleren Molekulargewicht von 5000 Da und Cₙ hydrophobe Ketten mit n ist 16 umfassen.

7. Pharmazeutische Zusammensetzung zur lokalen Verabreichung zur Verwendung bei der Diagnose von ophthalmischen Krankheiten umfassend ein Diagnostikum, wobei das Diagnostikum in einem pharmazeutischen Träger enthalten ist, der durch Polymer-Mizellen gebildet ist, die "Propf"-Typ-Copolymere von Polyaspartamid (PHEA) umfassen, die hydrophile Polyethylen-Glykol (PEG) Ketten mit einem mittleren Molekulargewicht umfassend zwischen 750 und 20000 Da und/oder hydrophobe Cₙ Alkyl-Ketten mit 4 ≤ n ≤ 20 beinhalten.

## Revendications

1. Compositions pharmaceutiques destinées au traitement de maladies ophtalmiques par administration locale, comprenant un ou plusieurs ingrédients actifs sélectionnés parmi des agents antimicrobiens, des agents anti-inflammatoires, des agents antiglaucomateux, des agents antiviraux, des agents antiangiogènes, des antioxydants, ledit principe actif étant inclus dans un vecteur pharmaceutique composé de micelles de polymères contenant des copolymères de type « greffé » de polyaspartamide (pHEA) contenant des chaînes hydrophiles de polyéthylène-glycol (PEG) ayant un poids moléculaire moyen compris entre 750 and 20 000 Da et/ou des chaînes hydrophobes d'alkyle Cₙ, où 4 ≤ n ≤ 20.

2. Compositions pharmaceutiques selon la revendication 1, comprenant en outre un ou plusieurs excipients acceptables du point de vue pharmaceutique pour administration ophtalmique.

3. Compositions pharmaceutiques selon la revendication 2, dans lesquelles les excipients sont sélectionnés parmi des bioadhésifs, des électrolytes (phosphates, chlorures), des agents isotonisants non ioniques, des agents tensioactifs, des antioxydants, des systèmes de tampon.

4. Compositions pharmaceutiques selon la revendication 1, dans lesquelles le poids moléculaire moyen des chaînes de polyéthylène-glycol est égal ou compris entre 2 000 et 10 000 Da et le nombre d'atomes de carbone dans les chaînes hydrophobes est de 12 ≤ n ≤ 18.

5. Compositions pharmaceutiques selon la revendication 1 ou 4, dans lesquelles les chaînes hydrophiles sont constituées de polyéthylène-glycol (PEG) d'un poids moléculaire moyen compris entre 750 et 20 000 Da et les chaînes lipophiles de chaînes aliphatiques choisies dans le groupe comprenant : butylamine, pentylamine, hexylamine, heptylamine, octylamine, décylamine, undécylamine, dodécylamine, tridécylamine, tétradécylamine, hexadéylamine, octadécylamine, didécylamine.

6. Compositions pharmaceutiques selon les revendications 1 à 5, dans lesquelles les copolymères comprennent du PEG d'un poids moléculaire moyen de 5 000 Da et des chaînes hydrophobes Cₙ dans lesquelles n est égal à 16.

7. Composition pharmaceutique pour administration locale destinée au diagnostic de maladies ophtalmiques, comprenant un agent de diagnostic, lequel agent de diagnostic est inclus dans un vecteur pharmaceutique composé de micelles de polymères contenant des copolymères de type « greffé » de polyaspartamide (PHEA) contenant des chaînes hydrophiles de polyéthylène-glycol (PEG) d'un poids moléculaire moyen compris entre 750 et 20 000 Da et/ou des chaînes hydrophobes d'alkyle Cₙ, où 4 ≤ n ≤ 20.
